# DEMANDE DE BREVET EUROPEEN

(11) **EP 1 880 683 A1**
(43) Date de publication de la demande: **23.01.2008**
(21) Numéro de dépôt: 06117350.6
(22) Date de dépôt: 18.07.2006
(51) Int. Cl.: A61B 17/16, A61C 1/14

(54) **Système d'accouplement pour instrument à main à usage dentaire ou chirurgical**

(71) Demandeur: Bien-Air Holding SA, 2504 Bienne (CH)
(72) Inventeur: Maitre, Luc, 2885 Epauvillers (CH)
(74) Mandataire: GLN

(57) **Abrégé**

L'invention concerne un système d'accouplement des arbres du mécanisme à pince aval et du mécanisme moteur amont d'un instrument à main, à usage dentaire ou chirurgical. Il comporte :
- un arbre creux (13) dont l'extrémité amont est conformée pour s'accoupler à l'extrémité aval (11) de l'arbre du mécanisme moteur et dont l'extrémité aval peut coulisser autour de l'extrémité amont (10) de l'arbre du mécanisme à pince,
- une goupille amovible (15) liant l'extrémité aval de l'arbre creux et l'extrémité amont de l'arbre du mécanisme à pince,
- un ressort (19) tendant à éloigner l'un de l'autre l'arbre creux et l'arbre du mécanisme à pince,
- deux fenêtres oblongues (14) diamétralement opposées pratiquées axialement dans l'extrémité aval de l'arbre creux et servant à limiter la course de la goupille de manière à définir, pour l'arbre creux et l'arbre du mécanisme à pince, une première position relative dans laquelle ils sont éloignés au maximum et une deuxième position relative dans laquelle ils sont rapprochés au maximum, et
- une douille (20) fixée sur l'extrémité aval de l'arbre creux et dont la longueur est telle que, uniquement dans ladite deuxième position, elle laisse apparaître la goupille afin d'autoriser son insertion ou son extraction qui permettent respectivement de connecter ou séparer le système d'accouplement et le mécanisme à pince.

## Description

La présente invention se rapporte au domaine des instruments médicaux. Elle concerne, plus particulièrement, un système d'accouplement pour un instrument à main destiné à faire tourner à grande vitesse un outil amovible dont l'extrémité constitue une fraise ou organe analogue utilisable dans les cabinets ou laboratoires dentaires ainsi qu'en microchirurgie.

Un instrument de ce type est décrit, par exemple, dans le document WO 2005/089666. Typiquement, la queue de l'outil est maintenue par une pince disposée à l'intérieur d'un arbre rotatif creux entraîné à son extrémité par un moteur électrique ou une turbine à air et monté par des roulements dans un fourreau tubulaire fixe. Le serrage et le desserrage de la pince se fait en tournant dans un sens ou l'autre un manchon monté rotatif sur le fourreau.

Un tel instrument est généralement réalisé en trois parties, à savoir un mécanisme à pince avec ses arbres mené et menant pour la fixation de l'outil, un mécanisme moteur pour l'entraînement en rotation du mécanisme à pince et un système d'accouplement du mécanisme à pince avec le mécanisme moteur.

Ces trois composants doivent pouvoir être aisément séparés les uns des autres afin de permettre au même mécanisme moteur d'actionner différents types de mécanisme à pince et d'autoriser le remplacement du système d'accouplement soumis à des sollicitations qui accélèrent son usure.

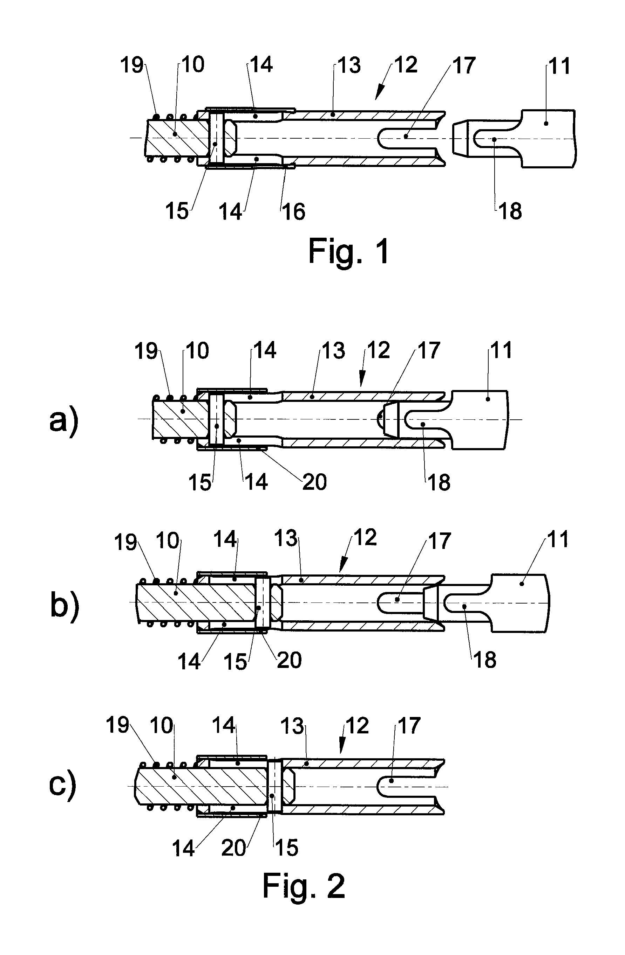

On se référera, tout d'abord, à la figure 1 qui montre, dans une réalisation de l'art antérieur, la manière dont les trois composants sont assemblés. Afin de faciliter la lecture, la présente description utilisera les mots « amont » et « aval » pour désigner respectivement l'extrémité de l'instrument à partir de laquelle l'outil est entraîné en rotation (partie droite du dessin) et son extrémité portant la pointe active de l'outil (partie gauche du dessin). De plus, le dessin ne montre pas les parties amont et aval de l'instrument car celles-ci peuvent être identiques à celles décrite dans le document WO 2005/089666 précité, auquel on pourra se référer pour comprendre la manière dont se fait l'entraînement de l'outil.

Sur la figure 1, on a représenté en 10 l'extrémité amont de l'arbre menant du mécanisme à pince, en 11 l'extrémité aval de l'arbre du mécanisme moteur et en 12 le système d'accouplement selon l'invention.

Celui-ci comporte un arbre creux 13 doté, à son extrémité aval, de deux fenêtres axiales oblongues 14 diamétralement opposées. L'extrémité amont 10 de l'arbre du mécanisme à pince est montée coulissante dans l'extrémité aval de l'arbre 13 et traversée par une goupille de connexion amovible 15 dont la course est limitée axialement par les deux fenêtres 14. Une douille fendue 16, clipsée autour de la portion de l'arbre 13 qui comporte ces fenêtres, assure le maintien en place de la goupille 15. Pour séparer l'arbre menant du mécanisme à pince du système d'accouplement, il est donc nécessaire de déclipser la douille 16 afin de faire apparaître la goupille 15 qui peut donc être retirée et permet ainsi de séparer les arbres 10 et 13.

L'extrémité amont de l'arbre 13 est dotée de deux encoches axiales oblongues 17 diamétralement opposées (une seule apparaissant au dessin) qui sont dimensionnées et conformées de manière à recevoir deux ailettes 18 (une seule apparaissant au dessin) qui terminent l'arbre 11 du mécanisme moteur. Selon une variante de réalisation, le nombre de paires d'encoches diamétralement opposées peut être plus élevé, afin de faciliter l'insertion des ailettes 18.

On notera, enfin, la présence d'un ressort 19 qui est disposé autour de l'arbre 10 et a pour effet de repousser l'arbre 13 vers l'amont. Grâce à ce ressort, même si le praticien n'a pas pris soin d'arrêter le moteur, l'arbre 13 a toujours la possibilité de se rétracter plus ou moins afin de faciliter la connexion du mécanisme moteur au système d'accouplement.

Si un tel mode de connexion du mécanisme moteur au système d'accouplement donne pleine satisfaction, il n'en est pas de même pour la connexion du système d'accouplement à l'arbre menant du mécanisme à pince. En effet, la solution décrite ci-dessus fait appel à une douille assez complexe dont la réalisation n'est pas aisée et dont le montage ou démontage nécessite un ouillage spécial. De plus, cette douille fendue clipsée ne garantit pas une tenue capable de résister à une connexion alors que le moteur est toujours en rotation.

La présente invention a notamment pour but de fournir un système d'accouplement exempt des inconvénients mentionnés ci-dessus.

De façon plus précise, l'invention concerne un système d'accouplement des arbres du mécanisme à pince aval et du mécanisme moteur amont d'un instrument à main, à usage dentaire ou chirurgical, caractérisé en ce qu'il comporte :
- un arbre creux dont l'extrémité amont est conformée pour s'accoupler à l'extrémité aval de l'arbre du mécanisme moteur et dont l'extrémité aval peut coulisser autour de l'extrémité amont de l'arbre du mécanisme à pince,
- une goupille amovible liant l'extrémité aval dudit arbre creux et l'extrémité amont de l'arbre dudit mécanisme à pince,
- un ressort tendant à éloigner l'un de l'autre ledit arbre creux et l'arbre du mécanisme à pince,
- deux fenêtres oblongues diamétralement opposées pratiquées axialement dans l'extrémité aval dudit arbre creux et servant à limiter la course de ladite goupille de manière à définir, pour ledit arbre creux et l'arbre du mécanisme à pince, une première position relative pour laquelle l'arbre creux et l'arbre du mécanisme à pince sont éloignés au maximum et une deuxième position relative pour laquelle ils sont rapprochés au maximum, et
- une douille fixée sur l'extrémité aval dudit arbre creux et dont la longueur est telle que, uniquement dans ladite deuxième position, elle laisse apparaître la goupille afin d'autoriser son insertion ou son extraction qui permettent respectivement de connecter ou séparer le système d'accouplement et le mécanisme à pince.

D'autres caractéristiques et avantages de la présente invention ressortiront de la description qui va suivre, faite en regard du dessin annexé dans lequel la figure 2 est une vue en coupe longitudinale d'un tel système d'accouplement, les éléments communs à la figure 1 illustrant l'art antérieur étant désignés par les mêmes numéros de référence.

La figure 2 montre en a, b et c le système selon l'invention respectivement :
- dans une première position extrême, pour laquelle l'extrémité amont 10 de l'arbre du mécanisme à pince est la moins engagée dans l'arbre creux 13 ;
- dans une position intermédiaire, lors de la connexion d'un moteur ; et
- dans une deuxième position extrême, pour laquelle l'extrémité amont 10 de l'arbre du mécanisme à pince est la plus engagée dans l'arbre creux 13.

Le système de la figure 2 présente, par rapport au système de la figure 1, les deux différences essentielles.

Premièrement, la douille amovible 16 est remplacée par une douille inamovible 20 avantageusement fixée par chassage sur l'extrémité aval de l'arbre creux 13.

Deuxièmement, cette douille inamovible 20 n'obture pas totalement les deux ouvertures oblongues 14. Sa longueur est telle que :
- dans la première position extrême (figure 2a), prise automatiquement, sous l'action du ressort 19, lorsque l'instrument est au repos ou au travail, elle couvre la goupille 15 ;
- dans la position intermédiaire (figure 2b), prise normalement sous l'effet de la force exercée vers l'aval lors de la connexion d'un moteur, elle couvre encore la goupille 15 qui ne risque pas ainsi de s'échapper ;
- dans la deuxième position extrême (figure 2c), prise lors de la connexion ou déconnexion au mécanisme à pince et nécessitant que l'opérateur exerce une poussée vers l'aval sensiblement plus forte que dans le cas d'une simple connexion du moteur, elle laisse apparaître la goupille 15 afin d'autoriser son insertion ou son extraction qui permettent respectivement de connecter ou séparer le système d'accouplement et le mécanisme à pince.

Ainsi est proposé un système d'accouplement qui facilite grandement les opérations de montage et démontage. Il suffit alors simplement, en effet, pour l'opérateur, de repousser manuellement au plus loin vers l'aval, en luttant contre le ressort 19, le tube creux 13 pour faire apparaître la goupille 15 qui peut être mise en place ou retirée. On notera aussi que la tenue de la douille 20 résiste, mieux qu'une douille fendue simplement clipsée, à une connexion lorsque le moteur est toujours en rotation, On remarquera enfin que la douille 20 est un simple tube dont la réalisation est particulièrement facilitée par rapport à celle de la douille fendue clipsée de l'art antérieur.

## Revendications

1. Système d'accouplement des arbres du mécanisme à pince aval et du mécanisme moteur amont d'un instrument à main, à usage dentaire ou chirurgical, **caractérisé en ce qu'**il comporte :
- un arbre creux (13) dont l'extrémité amont est conformée pour s'accoupler à l'extrémité aval (11) de l'arbre du mécanisme moteur et dont l'extrémité aval peut coulisser autour de l'extrémité amont (10) de l'arbre du mécanisme à pince,
- une goupille amovible (15) liant l'extrémité aval dudit arbre creux et l'extrémité amont de l'arbre dudit mécanisme à pince,
- un ressort (19) tendant à éloigner l'un de l'autre ledit arbre creux et l'arbre du mécanisme à pince,
- deux fenêtres oblongues (14) diamétralement opposées pratiquées axialement dans l'extrémité aval dudit arbre creux et servant à limiter la course de ladite goupille de manière à définir, pour ledit arbre creux et l'arbre du mécanisme à pince, une première position relative dans laquelle ils sont éloignés au maximum et une deuxième position relative dans laquelle ils sont rapprochés au maximum, et
- une douille (20) fixée sur l'extrémité aval dudit arbre creux et dont la longueur est telle que, uniquement dans ladite deuxième position, elle laisse apparaître la goupille afin d'autoriser son insertion ou son extraction qui permettent respectivement de connecter ou séparer le système d'accouplement et le mécanisme à pince.

2. Système d'accouplement selon la revendication 1, destiné à un arbre de mécanisme moteur se terminant par au moins une ailette (18), **caractérisé en ce que** l'extrémité amont dudit arbre creux (13) est dotée d'encoches oblongues (17) qui sont dimensionnées et conformées de manière à recevoir ladite ailette.
